Europäisches Patentamt

European Patent Office

Office européen des brevets

(11) Publication number: **0 255 340**
**A1**

(12) **EUROPEAN PATENT APPLICATION**

(21) Application number: **87306660.9**

(22) Date of filing: **28.07.87**

(51) Int. Cl.⁴: **C 12 N 15/00**
C 07 H 21/04, C 12 N 1/20,
A 01 H 1/00

(30) Priority: **28.07.86 US 889779**

(43) Date of publication of application:
**03.02.88 Bulletin 88/05**

(84) Designated Contracting States:
**AT BE CH DE ES FR GB GR IT LI LU NL SE**

(71) Applicant: **THE NATIONAL DEVELOPMENT CORPORATION LIMITED**
**Haddington Court Haddington Road**
**Dublin 4 (IE)**

(72) Inventor: **O'Gara, Fergal**
**36 Central Avenue**
**Bishopstown Cork (IE)**

(74) Representative: **Ruffles, Graham Keith et al**
**MARKS & CLERK 57-60 Lincoln's Inn Fields**
**London WC2A 3LS (GB)**

The applicant has filed a statement in accordance with Rule 28 (4) EPC (issue of a sample only to an expert). Accession number(s) of the deposit(s): NCIB 12293, NCIB 12294, NCIB 12295.

(54) **Dicarboxylic acid transport genes.**

(57) The invention relates to DNA fragments encoding genes which complement dicarboxylic acid transport mutants of Rhizobium species including Bradyrhizobium species and which confer increased nitrogen fixing ability on plants infected/inoculated with Rhizobium species, to plasmids encoding such DNA sequences and to bacterial hosts carrying the said DNA fragments or plasmids. The invention further relates to a method of increasing dicarboxylic acid transport in Rhizobium species and to a method of increasing the nitrogen fixing ability of plants by infecting or inoculating the plants with bacterial hosts containing the above described DNA fragments.

EP 0 255 340 A1

**Description**

"Dicarboxylic Acid Transport Genes"

The present invention relates to the cloning of dicarboxylic acid transport (dct) genes, to DNA sequences encoding dicarboxylic acid transport genes and to the use of such DNA sequences in the improvement of bacterial strains capable of carrying out nitrogen fixation.

The fixation of molecular nitrogen from the air, i.e. its reduction to yield ammonia and other biologically useful forms of nitrogen is an extremely important process in the biosphere. However, it can be carried out by only a limited number of organisms. The majority of organisms must obtain nitrogen in a combined form such as nitrate, ammonia or amino acids. Most leguminous plants e.g. clover and alfalfa can fix atmospheric nitrogen, but the process requires the symbiotic action of the host plant and bacteria present in the root nodules of the plant. The bacteria involved in nitrogen fixation belong mainly to the genus Rhizobium, and these bacteria enter the cortical parenchyma of the root where they give rise to nodules. The nodules are highly organized structures with membraneous sacks containing colonies of bacteria. Supplied with carbon compounds from photosynthesis by the plant, the bacteria fix nitrogen, producing ammonia which is provided to the plant for amino acid biosynthesis, thus aiding protein formation in the plant. Leguminous plants can therefore be grown without synthetic nitrogen fertilizers which represents a considerable financial saving for the farmer since it allows for protein production on a cost effective basis.

The process of nitrogen fixation is the basis for the use of clover and alfalfa in crop rotation, since the growth of such plants increases the utilizable nitrogen in the soil.

Legume seeds which have been coated with Rhizobium species are currently available commercially. This allows for the formation of nitrogen fixing root nodules to be formed as soon as the seeds germinate. The seeds are coated with naturally occurring strains which have been isolated from soil and selected for their efficiency in fixing nitrogen and their ability to compete with other bacterial strains in the soil.

Evidence indicates that the nitrogen-fixing capacity of the Rhizobium-legume symbosis is influenced by the amount of photosynthate available to the bacteria in the root nodule (Hardy and Havelka, (1975) In: Nutman P.S. (ed) International biological programme : symbotic nitrogen fixation in plants. Cambridge University Press, New York, pp 421-439; Havelka and Hardy (1976) In : Newton W.E., Nyman, C.Y. (eds) Proceedings of the first international symposium on nitrogen fixation. Washington State University Press, Pullman, Washington, pp 456-475; and Ryle et al (1979) J. Exp. Bot. 30 : 135-144). It is widely thought that one of the key limiting steps in symbiotic nitrogen-fixation is the availability of the carbon (i.e. energy) source made available to the bacteria.

The inventors of the present invention do not agree fully with this hypothesis but believe that the limiting factor may not be the availability of a carbon source but rather the transport of the carbon source into the bacteria in the root nodule.

Tricarboxylic acid (TCA) cycle intermediates have been implicated as the major carbon source supplied to bacteroids (i.e. the differentiated form of bacteria which fixes nitrogen in the root nodule) in pea and clover symbiosis. The evidence for this is based on the fact that dicarboxylic acid transport mutants of Rhizobium leguminosarum (Finan et al (1983) J.Bacteriol 148, 193-202; Glenn and Brewin (1981) J.Gen. Microbiol. 126, 237-242) and R. Trifolii (Ronson et al (1981) PNAS (USA) 78, 4284-4288) form ineffective nodules despite being able to utilize other carbon sources. In R. meliloti, a functional TCA cycle is necessary to support nitrogen-fixation in the bacteroid as shown by the fact that mutants defective in succinate dehydrogenase (Gardiol et al (1982) J. Bacteriol. 151 1621-1623) and alpha-ketoglutarate dehydrogenase (Duncan and Fraenkel (1979) J. Bacteriol. 37 415-419) form ineffective nodules.

The strain formerly known as Rhizobium japonicum has been oficially re-classified as Bradyrhizobium japonicum and references to that strain in this specification have been amended accordingly. The term "Rhizobium species" as used herein includes both Rhizobium and Bradyrhizobium species.

It is an object of this invention to increase the nitrogen fixing ability of leguminous plants.

The present invention relates to a fragment of DNA encoding genes which complement dicarboxylic acid transport mutants of Rhizobium species, and which confer increased nitrogen fixing ability on plants infected/inoculated with Rhizobium species.

The invention also relates to plasmids carrying a DNA fragment encoding genes which complement the dicarboxylic acid transport mutants of Rhizobium species, and which confer increased nitrogen fixing ability on plants infected/inoculated with Rhizobium species.

In particular, the invention relates to plasmids deposited with the National Collection of Industrial Bacteria, Torry Research Station, Aberdeen, Scotland, on 22nd July 1986, under the accession numbers NCIB 12293, NCIB 12294 and NCIB 12295, isogenic derivatives thereof, plasmids which are substantially similar thereto in the region of DNA encoding genes which complement dicarboxylic acid transport mutants of Rhizobium species, particularly plasmids having at least 80% homology with the said DNA region; and DNA in such plasmids encoding genes which complement dicarboxylic acid transport mutants of Rhizobium species.

The invention also relates to bacterial hosts containing DNA fragments as defined above or one or more of the plasmids as defined above. In particular, the invention provides bacterial hosts containing plasmids having the accession numbers NCIB 12293, NCIB 12294 and NCIB 12295.

The invention especially relates to all of the above-defined materials in biologically pure form.

The invention also provides a method of increasing dicarboxylic acid transport in Rhizobium species by transferring into the said species a DNA fragment encoding genes which complement dicarboxylic acid transport mutants of Rhizobium species, or a plasmid carrying such a DNA fragment.

The invention further provides a method of increasing the nitrogen fixing ability of plants by infecting/inoculating the said plants with a bacterial host carrying a DNA fragment encoding genes which complement the dicarboxylic acid transport mutants of Rhizobium species, or a bacterial host transformed with a plasmid carrying such a DNA fragment.

In a still further aspect of the invention there are provided seeds of plants which have been coated with bacterial hosts as defined above.

The invention will now be described with reference to the accompanying drawings in which:-

Figure I shows an analysis of plasmid 4:46 by agarose gel electrophoresis. Track I:- BglII, Track 2:- SalI, Track 3:- BglII/SalI, Track 4:- PstI, Track 5:- ClaI, Track 6:- HindIII,.

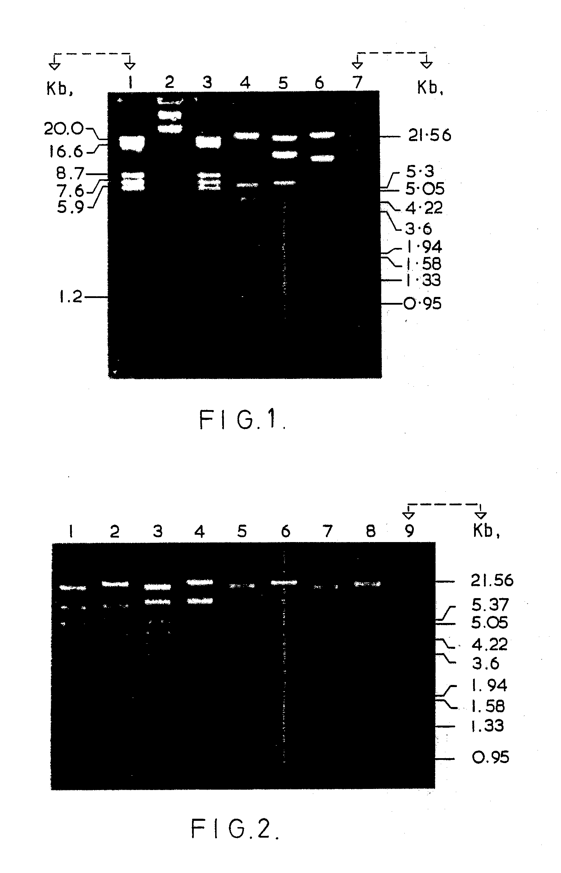

Figure 2 shows an analysis of plasmid 4:46 by agarose gel electrophoresis. Track I:- BglII/EcoRI, Track 2:- EcoRI, Track 3:- BglII/HindII, Track 4:- HindIII, Track 5:- BglII/PstI, Track 6:- PstI, Track 7: BglII/SmaI, Track 8:- SmaI.

Figure 3 shows the $CO_2$ trap used in the succinate metabolism assay.

Figure 4 is a graph showing succinate uptake by the R. japonicum strains CJI, CJI (MC5), CJI (4:46) and CJI (4:46:Tn5).

Figure 5 shows nitrogenase activity as a function of malate and oxygen concentrations for the B. japonicum wild type strain CJI and the strain CJI )pRK290:4:46).

## ISOLATION OF DCT MUTANTS OF R. MELILOTI

Isolation of dct mutants of R. meliloti was as described in Bolton et al Arch. Microbiol. (1986) 144 142-146.

## Succinate Uptake Assay.

Succinate uptake analysis showed that both NTG mutants (R. meliloti CMIO-19) and the Tn5 mutant (R. meliloti CM20) were unable to accumulate $^{14}$C- labelled succinate indicating a defect in their dicarboxylic acid transport system (Bolton et al Arch. Microbiol. (1986) 144 142-146).

Verification of the Tn5 associated nature of the mutation was obtained using coinheritance studies during gene transfer experiments with the wild type strain. The R68 derived plasmid pJB3JI kanamycin sensitive (Kans) (Brewin et al. (1980) J. Gen. Microbiol. 120 413-420) capable of promoting chromosome transfer in R. meliloti (Kondorsoi et al. (1977) Nature 268 525-527) was conjugated into R. meliloti CM20 on MSY media at 30°C for 24 h. R. meliloti CM20 pJB3JI exconjugants were selected on MSY Rif Tc plates. pJB3JI was subsequently transferred from R. meliloti CM20 to the wild type R. meliloti strain. Tn5 containing exconjugants were selected on MSY Kan and subsequently the succinate phenotype was checked on minimal medium containing succinate (0.1%).

## SYMBIOTIC PROPERTIES

The R. meloliti dct mutants were able to nodulate Alfalfa plants but the nodules formed were unable to fix nitrogen (Bolton et al. Arch. Microbiol. (1986) 144 142-146).

## Nitrogenase activity in nodules

Assay of nitrogenase activity in nodules was as described in Bolton et al. Arch. Microbiol. 144 142-46.

## CLONING OF DCT GENES IN R. MELILOTI

The cloning of dct genes from R. meliloti, plasmid DNA isolation and restriction and the analysis of the effect of dct gene dosage on succinate transport in R. meliloti were as described in Bolton et al. Arch. Microbiol. 144 142-146.

## IDENTIFICATION OF PLASMIDS 4:46 AND MC5 CONTAINING DCT GENES

## Plasmid 4:46

R. meliloti CMI2 was complemented using the pRK290 BglII gene bank of R. meliloti F34 contained in E. coli HBIOI (obtained from Prof. D.R. Helsinki of the University of California at San Diego). This gene bank is maintained on 28 LB plates containing 10 µg/ml of Tc with 49 clones per plate.

R. meliloti CMI2 was grown in 50 mls of MSY while the mobilizing strain pRK2013 required to mobilize pRK290 was grown in 50 ml of LB broth containing 25 µg/ml of Kan to maintain pRK2013.

The HBIOI R. meliloti F34 gene bank containing the 2013 mobilizing plasmid was mated with R. meliloti CMI2 on MSY plates for 48 hours at 30°C. The mating mix was then transferred to exconjugant selection plates, i.e., MSY rif Tc (100 µg/ml rif selecting for the mutant strain while 5 µg/ml Tc selects for pRK290 plasmid transfer) and incubated at 30°C for up to 5 days. Exconjugants were then transferred to succinate $NH_4$ selective plates. Clones which grew on these plates presumably obtained complementing DNA from the gene bank. Plasmid DNA for study was obtained from the corresponding HBIOI gene bank clones on LB Tc plates. The complementing plasmid is referred to as 4:46. A flow diagram of the complementation procedure is as follows:-

Flow diagram of Complementation Procedure

50 mls of an overnight 2013 culture grown in LB$_{Kan}$ (25 µg/ml) centrifuged at 7,000 rpm for 5 min at 4°C.

↓

2013 pellet resuspended in 3 ml of ringers and LB plates spread with 0.1 ml.

↓

HB101 pRK290 gene bank transferred to seeded plates and incubated overnight at 37°C.

↓

Exconjugants selected on LB$_{Tc\ Kan}$

↓

50 ml of a 24 hr, mutant culture centrifuged at 7,000 rpm for 5 mins, at 4°C. Pellet washed in ringers and recentrifuged.

↓

Mutant pellet resuspended in 3 ml of ringers and MSY plates spread with 0.1 ml.

↓

Gene bank with 2013 transferred onto seeded MSY plates and incubated at 30°C for 48 hours.

↓

Mating transferred to MSY rif Tc to select exconjugants and incubated at 30°C for 4-5 days.

↓

Exconjugants transferred to Suc NH$_4$ plates and incubated at 30°C for 4-5 days.

↓

Suc NH$_4$ plates examined for complemented mutant growth.

↓

Corresponding gene bank clone used for plasmid isolation.

R. meliloti CMI2 containing plasmid 4:46 was deposited with the National Collection of Industrial Bacteria, Torry Research Station, Aberdeen, Scotland under the accession number I2294.

Plasmid MC5

Plasmid MC5 was identified by an essentially similar procedure to the one described above except that a pLAFRI gene bank of R. meliloti F34 (also obtained form Prof. D.R. Helsinki) was used as the source of the complementing plasmids.

R. meloliti CMI2 containing plasmid MC5 was deposited with the National Collection of Industrial Bacteria, Torry Research Station, Aberdeen, Scotland under the accession number I2293.

Analysis of Plasmids 4:46 and MC5

An analysis of plasmids 4:46 and MC5 was carried out using a variety of restriction enzymes. The restriction data is as follows and as shown in Figures I and 2:-

## Plasmid 4:46

| Restriction Enzyme | No. of Fragments |
|---|---|
| BglII | 6 |
| ClaI | 4 |
| EcoRI | 7 |
| HindIII | 4 |
| PstI | 9 |
| SalI | 1 |
| SmaI | 8 |
| BglII/EcoRI | 8 |

BglII/HindIII        10
BglII/PstI        12
BglII/SalI        6
BglII/SmaI        11

## Plasmid MC5

| Restriction Enzyme | No. of Fragments |
|---|---|
| BamHI | 3 |
| BglII | 7 |
| EcoRI | 7 |
| PstI | 5 |
| HindIII | 4 |

## Restriction Data of Plasmid 4:46

| Restriction Enzyme | Fragment sizes (kb) |
|---|---|
| BglII | 20.00, 16.60, 8.70, 7.60, 5.90, 1.20 |
| ClaI | 21.00, 13.40, 6.75, 3.45 |
| EcoRI | 21.55, 12.40, 6.15, 5.50, 3.20, 1.55, 1.45 |
| HindIII | 21.00, 12.90, 5.35, 1.75 |
| PstI | 21.00, 5.50, 3.90, 3.20, 2.55, 1.65, 1.55, 1.30, 1.05 |
| SmaI | 18.20, 7.25, 5.50, 5.00, 4.15, 3.35, 2.70, 2.50 |
| BglII/EcoRI | 19.00, 9.35, 5.75, 5.25, 3.80, 2.30, 1.20, 0.85 |
| BglII/HindIII | 20.00, 11.40, 6.05, 4.20*, 2.70, 1.70, 1.20*, 0.85 |
| BglII/PstII | 20.00, 5.50, 3.40, 3.00*, 2.50, 2.05, 1.65*, 1.50, 1.20, 1.05 |
| BgII/SmaI | 15.80, 5.0, 4.20 3.75, 3.40, 3.20, 2.75, 2.50, 1.60*, 7.20 |

*These bands are doublets.

# Restriction Data of Plasmid pMC5

| Restriction Enzyme | Fragment Sizes (kb) |
|---|---|
| EcoRI | 21.60, 12.75, 3.05, 2.80, 2.60, 1.20, 0.85 |
| BglII | 19.60, 8.90, 8.00, 3.60, 1.95, 1.65, 1.15 |

Hybridisation of Plasmid pMC5

Plasmid pMC5 contains sequences homologous to R. leguminosarum dct region. The region of homology has been localised to a 8.9 kb BglII fragment.

TRANSFER OF PLASMID 4:46 TO R. MELILOTI CM2

The procedure used was essentially similar to that described for the complementation experiments except that the donor strain was E. coli HBIOI (4:46), the mobilizing strain was E. coli HBIOI (pRK2OI3) and the recipient strain was R. meliloti CM2.

TRANSFER OF PLASMIDS 4:46 AND MC5 TO B. JAPONICUM CJI

Depending on which plasmid was to be transferred either E. coli HBIOI (4:46) or E. coli HBIOI (MC5) served as the donor strain. In both cases the mobilising strain was E. coli HBIOI (pRK2OI3) and the recipient strain was B. japonicum CJI. The donor strains were grown in LB medium containing IO μg/ml tetracycline, the mobilising strain in LB medium containing 25 μg/ml kanamycin and the recipient strain in MSY medium. The mating was preformed on MSY plates for 48h at 30°C. The mating mix was then transferred to the exconjugant selection plates, i.e. MSY plates containing IOO μg/ml spectinomycin (selecting for B. japonicum CJI) and I2O μg/ml tetracycline (selecting for transfer of plasmids 4:46 or MC5) and incubated at 30°C for 7 days.

The frequency of tetracycline transfer was $8.7 \times 10^{-5}$. The vector plasmid pRK29O was also mobilized into B. japonicium CJI and served as a control in subsequent experiments.

R. japonicum CJI containing plasmid MC5 was deposited with the National Collection of Industrial Bacteria, Torry Research Station, Aberdeen, Scotland under the accession number I2295.

TRANSFER OF PLASMID pRK29O:4:46 TO R. LEGUMINOSARUM CLIOO.

Plasmid p446 was transferred to R. leguminosarum CLIOO using plasmid pRK2OI3 as a helper plasmid. The donor strain E. coli KMBLII64 (p446) was grown in LB medium containing IO μg/ml tetracycline, the mobilising strain E. coli KMBLII64 (pRK2OI3) in LB medium containing 25 μg/ml kanamycin and the recipient strain R. leguminosarum CLIOO in MSY medium. The mating was performed on MSY plates for 48 h at 30°C.

The mating mix was then transferred to the selection plates, i.e. MSY plates containing 5O μg/ml streptomycin (selecting for R. leguminosarum CLIOO) and 2O μg/ml tetracycline (selecting for transfer of plasmid p446) and incubated at 30°C for 4 days.

INCREASED TRANSPORT ACTIVITY IN R. MELILOTI, B. JAPONICUM AND R. LEGUMINOSARUM CLIOO

Transport Assay

Log phase cells grown in 2OO ml succinate $NH_4^+$ were centrifuged at 8,OOO rpm for 5 minutes at 4°C, and washed twice in Rhizobium salts buffer pH 6.8. The cells were resuspended in 3 ml transport assay solution which consisted of Rhizobium salts IO ml/l, MOPS IO.4 g/l, ammonium sulphate I g/l at pH 6.8. Cell suspension was starved at 3O° for three hours prior to the transport assay.

In a typical assay, I ml of cells was added to two mls reaction mix in a sarsted tube. Reaction mix contained transport assay solution, 2O μM'Cold' succinate, I μCi 'Hot' succinate, pH 6.8 (specific activity II9.7 mCi/mM). Assay mixture was vortexed prior to removal of first sample. IOO μl samples were removed in duplicate at one minute intervals for ten minutes using a micropipette (pipetman p2OO). Samples were filtered using O.45 Gelman nitrocellulose filters on a millipore 3O25 sampling minifold. Filtered cells were immediately washed twice with 2 ml of ice cold Rhizobium salts containing I mM sodium azide. Washed cells and filters were placed in scintillation vials containing 6 ml of scintillation fluid. Vials were vortexed well and read on a Backman LS 7,OOO scintillation counter for IO minutes using program 3. Background counts were obtained by reading vials containing just scintillation fluid for I minute on program 3.

Non specific binding of $^{14}$C-labeled substrate to the bacterial cells was determined by using toluene-treated cells (2%) in a capped sarsted, mixing vigorously and placing in a shaking incubator for 30 minutes at 30°C before assay.

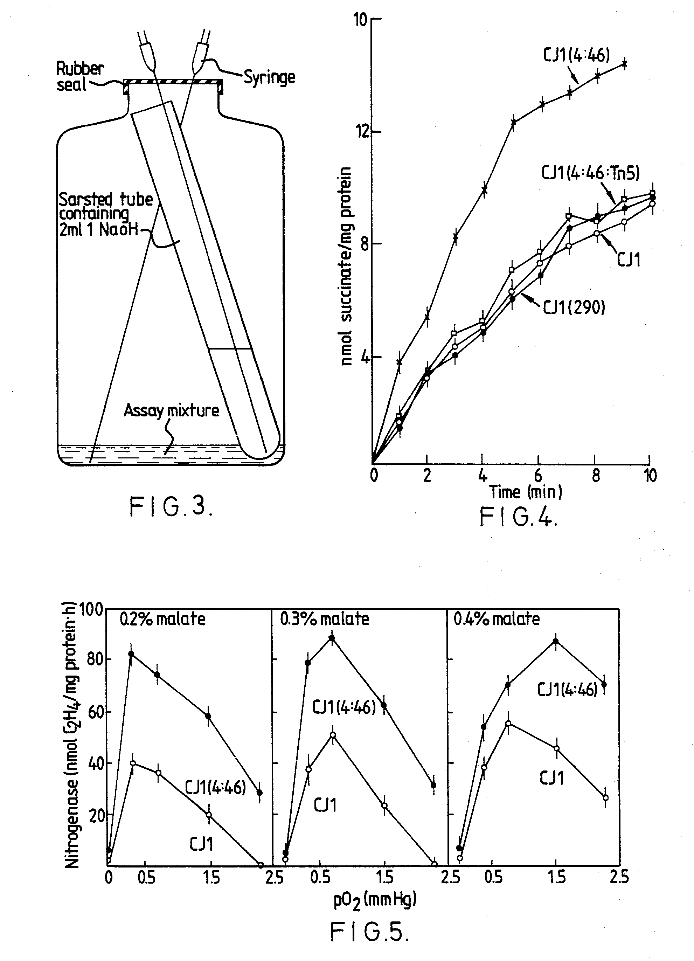

Assay for release of $^{14}CO_2$ from assay mixture was carried out using the $CO_2$ 'trap' illustrated in Figure 3. 100 µl samples were assayed in duplicate at time 1, 5, 10, 20, 40, 80 minutes. Samples were added directly to scintillation fluid, vortexed and counted for 10 minutes on program 3.

Protein Assay

Protein concentrations were determined by the method of Lowry et al. (1951) J.Biol.Chem. 193 (265-275).

Reagents:
    1) 3% $Na_2CO_3$ in 0.1N NaOH
    2) Alkaline Copper Reagent:-
2 ml of 2% $CuSO_45H_2O$ and 2 ml of 4% sodium tartarate made up to 100 ml in a volumeric flask using reagent 1.
    3) Folin Ciocalteau Reagent:-
1 part was diluted with two parts water just before use and protected from light with aluminium foil.
    4) Standards:-
Crystalline bovine serum albumin at 0, 30, 60, 90, 120, 150 g ml$^{-1}$ made up in same solvent as sample being assayed.

Procedure

To a sample or standard containing 10 - 200 µg protein in 0.5 ml in a test tube, 5 ml alkaline copper reagent was added, mixed well and allowed to stand at room temperature for at least 10 minutes, 0.5 ml diluted Folin-Ciocalteau reagent was added very rapidly and mixed within a second or two in a vortex. The mixture was allowed to stand for 30 minutes and extinction at 655 nm (E655) was measured. A standard curve relating E655 to mg protein per assay tube was drawn and the protein content of the unknown sample was estimated as mg protein assayed. Whole cells were solubilized by boiling at 80°C for 2-3 hours in 25 mls of 0.3 N NaOH prior to assay.

Succinate uptake was expressed as nmoles of succinate/mg protein and plotted against time. Results for R. meliloti strains are shown in Table I and results for B. japonicum strains are shown in Figure 4.

### TABLE 1: Succinate Uptake (nmoles succinate/mg protein)

| Time (mins) | CM2 | CM2( pRK 290:4:46) |
|---|---|---|
| 0 | 0.0 | 0.0 |
| 1 | 6.0 | 10.0 |
| 2 | 12.0 | 19.0 |
| 3 | 18.0 | 27.0 |
| 4 | 24.0 | 35.0 |
| 5 | 30.0 | 42.0 |
| 6 | 32.0 | 49.0 |
| 7 | 35.0 | 57.0 |
| 8 | 42.0 | 66.0 |
| 9 | 45.0 | 75.0 |
| 10 | 48.0 | 79.0 |

Table 1 shows succinate uptake by both the wild-type R. meliloti CM2 strain and the R. meliloti CM2 (4:46) strain.

The succinate uptake rate of B. japonicum CJI (pRK290:4:46) was compared with that of B. japonicum CJI during growth on succinate $NH_4^+$ minimal medium. A time course study (Fig. 4) revealed that the uptake of $^{14}$C-labelled succinate was about two fold higher in the strain containing plasmid pRK290:4:46 (2.8 nmol/mg protein.min) compared to B. japonicum CJI (1.4 nmol/mg protein.min). In both strains the pattern of uptake was similar and linear for approximately the first seven minutes. When a Tn5 containing derivative of pRK290:4:46 unable to complement the R. meliloti Dct– strain CMI2 was mobilised into B. japonicum CJI, no increase in

succinate uptake activity was observed in the resultant strain compared to B. japonicum CJI (Fig. 4). These studies indicate that the enhanced succinate uptake activity exhibited by B. japonicum CJI (pRK290:4:46) is a consequence of the expression of the introduced R. meliloti gene(s) in B. japonicum. In addition, it would appear that the same locus is responsible for both the Dct complementing phenotype and the increased succinate uptake activity.

Regulation of succinate uptake in B. japonicum strain CJI (pRK290:4:46)

Since the introduced R. meliloti gene(s) could be expressed in B. japonicum, the regulation of these genes was next investigated in the new background. The strains B. japonicum CJI and CJI (pRK290:4:46) were grown in minimal media containing a variety of different carbon sources and the specific growth rates determined (Table 2). An increase in the specific growth rate of B. japonicum CJI (pRK290:4:46) compared to that of strain CJI was only observed during growth on dicarboxylic acids (fumarate, malate or succinate). No significant difference was evident in the specific growth rates on other carbon sources. These results indicate that plasmid pRK290:4:46 specifically affects the utilisation of dicarboxylic acids.

The pattern of inhibition of succinate transport by metabolic inhibitors in the hybrid strain CJI (pRK290:4:46) was investigated and found not to differ from that in the wild type strain CJI. Sodium azide or sodium cyanide at a concentration of IO μM inhibited succinate uptake activities by over 80% in both strains.

TABLE 2: Growth characteristics of <u>dct</u> modified <u>B. japonicum</u> strains under aerobic conditions[a]

| Carbon Source | Specific growth rate ($h^{-1}$) | | |
|---|---|---|---|
| | CJ1 | CJ(pRK290) | CJ(pRK290:4:46) |
| Succinate | 0.072 | 0.069 | 0.103 |
| Fumarate | 0.070 | 0.066 | 0.091 |
| Malate | 0.070 | 0.064 | 0.092 |
| Gluconate | 0.052 | 0.050 | 0.054 |
| Arabinose | 0.050 | 0.047 | 0.051 |
| Pyruvate | 0.050 | 0.050 | 0.049 |
| Glutamate | 0.049 | 0.046 | 0.049 |
| Mannitol | 0.043 | 0.039 | 0.041 |
| Glucose | 0.035 | 0.033 | 0.036 |

[a]The cultures were grown aerobically as described in the test. The nitrogen source in all cases was $(NH_4)_2 SO_2$ (0.1% w/v).

An examination of the succinate uptake activities of the hybrid B. japonicum strains on minimal media containing various carbon sources showed that the highest rates of succinate uptake were obtained in cells grown on dicarboxylic acids (Table 3). This was similar to the pattern of succinate uptake exhibited by the wild type strain. Growth on other carbon compounds resulted in lower succinate uptake rates, clearly demonstrating that dicarboxylic acids are necessary for inducing maximum expression of the Dct system in both the wild type and modified strains. An investigation of the regulation of the Dct system by other carbon sources showed that glutamate, mannitol and arabinose did not significantly affect the transport rate in either strain CJI and CJI (pRK290:4:46) (Table 3). B. japonicum strain CJI containing the Tn5 derivative of plasmid pRK290:4:46 behaved very similarly to the parent strain CJI with respect to its succinate uptake capabilities (Table 3). These results demonstrate clearly that the expression of the R. meliloti gene(s) present on plasmid pRK290:4:46 results in a two-fold increase in the succinate uptake activity of the modified strain. Furthermore, this transport modification in the strain does not appear to affect the regulation of succinate uptake by either

8

metabolic inhibitors or other carbon sources compared to the wild type strain.

TABLE 3 : Regulation of succinate uptake activity in <u>dct</u> modified
<u>B</u>. <u>japonicum</u> strains under aerobic conditions

| Carbon Source | Succinate uptake activity (nmol/mg protein.min)[a] | | |
|---|---|---|---|
| | CJ1 | CJ1(pRK290:4:46) | CJ1pRK290:4:46:TN=n5) |
| Succinate | 1.43 $\pm$ 0.21(100)[b] | 2.82 $\pm$ 0.14(100) | 1.39$\pm$ 0.18(100) |
| Fumarate | 1.33 $\pm$ 0.26(93) | 2.62 $\pm$ 0.11(93) | ---[c] |
| Malate | 1.31 $\pm$ 0.24(92) | 2.68 $\pm$ 0.13(95) | 1.36 $\pm$ 0.14(98) |
| Gluconate | 0.54 $\pm$ 0.18(38) | 1.02 $\pm$ 0.18(36) | 0.62 $\pm$ 0.17(45) |
| Pyruvate | 0.50 $\pm$ 0.10(35) | 0.96 $\pm$ 0.09(34) | --- |
| Glutamate | 0.56 $\pm$ 0.17(39) | 1.16 $\pm$ 0.14(41) | 0.58 $\pm$ 0.09(42) |
| Mannitol | 0.49 $\pm$ 0.11(34) | 0.79 $\pm$ 0.19(28) | --- |
| Arabinose | 0.46 $\pm$ 0.10(32) | 0.95 $\pm$ 0.18(34) | 0.41 $\pm$ 0.08(30) |
| Ribose | 0.43 $\pm$ 0.13(30) | 0.90 $\pm$ 0.04(32) | --- |
| Succinate + Arabinose | 1.28 $\pm$ 0.24(90) | 2.71 $\pm$ 0.19(96) | 1.32 $\pm$ 0.18(95) |
| Succinate + glutamate | 1.36 $\pm$ 0.21(96) | 2.76 $\pm$ 0.16(98) | 1.28 $\pm$ 0.19(92) |
| Succinate + mannitol | 1.35 $\pm$ 0.23(95) | 2.76 $\pm$ 0.08(98) | 1.25 $\pm$ 0.14(90) |

[a]The cultures were grown aerobically as described in the test. The
nitrogen source in all cases was $(NH_4)_2SO_2$ (0.1% w/v). Succinate
uptake activity was determined using $^{14}C$-labelled succinate as
described in the text.

[b]Each value is the mean of three replicates and is associated with
its SE. The percentages of succinate uptake relative to the rates
obtained by cells when grown on succinate alone are shown in
parenthesis.

[c]activity not determined.

0 255 340

<u>Succinate Uptake in R. leguminosarum CLIOO (p446)</u>

As shown in Table 4 succinate uptake activity was significantly higher in the R. leguminosarum CLIOO strain carrying the plasmid pRK29O:4:46 than in the same strain carrying the pRK29O plasmid or carrying no plasmid at all.

TABLE 4:    Increase in Succinate Uptake Activity of  <u>R. leguminosarum</u> <u>CL100</u> by plasmid p 446

| Strain | Succinate uptake (nmol/min/mg protein) |
|---|---|
| <u>R. leguminosarum</u> CL100 | 26.2 |
| <u>R. leguminosarum</u> CL100 (pRK290) | 19.4 |
| <u>R. leguminosarum</u> CL100 (pRK290:4:46) | 38.2 |

<u>NITROGEN FIXATION IN R. MELILOTI CARRYING PLASMID pRK 290:4:46</u>

R. meliloti 4I and its derivatives were tested for their symbiotic properties on alfalfa. Nitrogen fixation rates were measured as described above and the results are shown in Table 5. An uninoculated control and R. meliloti CMI2 which has a dct− phenotype did not fix nitrogen. R. meliloti strains carrying the pRK29O:4:46 plasmid fixed more than twice as much nitrogen as the wild-type R. meliloti strain, indicating that an increase in the copy number of dct genes leads to an increase in nitrogen fixation.

TABLE 5: Increase in Nitrogen Fixation Rates by Gene Dosage effect with plasmid pRK290:4:46

| <u>Strain</u> | <u>Phenotype</u> | Nitrogen Fixation* <u>(n moles ethylene/hr/plant)</u> |
|---|---|---|
| <u>R. meliloti</u> CM2 | Dct$^+$ | 230 |
| <u>R. meliloti</u> CM2(4:46) | Dct$^+$ | 528 |
| <u>R. meliloti</u> CM12(4:46) | Dct$^+$ | 621 |
| <u>R. meliloti</u> CM12 | Dct$^-$ | 0 |
| Uninoculated control | − | 0 |

*Activities were determined 4 weeks following germination and inoculation of alfalfa seedlings.

Free-living Nitrogenase Activity of B. japonicium strain CJI (pRK290:4:46)

Nitrogenase activity was determined in cell suspensions of B. japonicium by the acetylene reduction technique described by O'Gara and Shanmugan (Biochem. Biophys. Acta. 437 3l3-327, (l977)). Cultures were grown in minimal salts media containing O.4% (w/v) malate and O.l% (w/v) glutamate and an oxygen concentration of O.76 mm Hg (standard nitrogenase induction condition), unless otherwise stated.

Having established that the presence of plasmid pRK290:4:46 in B. japonicum CJI results in an increased succinate uptake capability, the possible effect on this enhanced uptake activity on free-living nitrogenase activity under different growth conditions was next investigated. The concentrations of the dicarboxylic acid (O.2% to O.4% w/v malate) and oxygen (O.38 to l.52 mm Hg) provided during the nitrogenase induction assay were varied. Under all conditions tested, B. japonicum CJI (pRK290:4:46) exhibited higher acetylene reduction activities over the strain without plasmid pRK290:4:46 (Fig. 5). In some instances (e.g. O.4% malate and l.52 mm Hg $pO_2$ or O.2% malate and O.76 mm Hg $pO_2$) the increase was as high as 90%. The maximum level of nitrogenase activity induced by strain CJI (pRK:290:4:46) was 60% higher than the maximum level induced by strain CJI. It was also observed that strain CJI (pRK290:4:46) exhibited maximum levels of nitrogenase at a higher oxygen concentration than strain CJI (l.52 mm Hg $pO_2$ as opposed to O.76 mm Hg $pO_2$).

The succinate uptake activities of the two strains under free-living nitrogen fixing conditions were determined to see if any correlation existed between succinate uptake and nitrogenase activities. The results obtained indicate that, for both strains, maximal succinate uptake and nitrogenase activities were obtained under the same conditions (Table 6). These results suggest that the enhanced nitrogenase activity found in strain CJI (pRK290:4:46) is linked to the increased uptake activity associated with this strain.

TABLE 6: Succinate uptake and nitrogenase activities in B. japonicum cultures under free-living microaerobic conditions.

| Strain | Malate Conc. (w/v)[a] | Succinate Uptake Act. (nmol/mg prot.min)[d] | Nitrogenase Act. (nmol/mg prot.h)[c] |
|---|---|---|---|
| CJI | 0.2% | 1.29 ± 0.08 | 38.2 ± 3.1 |
| CJI(4:46) | | 2.59 ± 0.4 | 74.1 ± 2.9 |
| CJI | 0.3% | 1.09 ± 0.15 | 51.1 ± 2.1 |
| CJI(4:46) | | 2.86 ± 0.3 | 88.1 ± 1.7 |
| CJI | 0.4% | 1.46 ± 0.1 | 54.7 ± 4.6 |
| CJI(:4:46) | | 1.52 ± 0.16 | 71.2 ± 4.1 |
| CJI | 0.4%[b] | 1.48 ± 0.2 | 47.2 ± 2.1 |
| CJI(4:46) | | 2.98 + 0.7 | 88.6 ± 3.2 |

[a]The cultures were grown under microaerophilic conditions as described in the text with glutamate (0.1% w/v) as the nitrogen source, varying concentrations of malate as the carbon source and an oxygen concentration of 0.76 mm Hg.

[b]The cultures were grown in malate (0.4% w/v), glutamate (0.1% w/v) and an oxygen concentration of 1.52 mm Hg.

[c]The cell yield (mg protein/ml) of the cultures was determined 150 h after inoculation.

[d]Succinate uptake was measured approximately 50 h after inoculation.

Each value is the mean of at least three replicates and is associated with its SE.

The present invention has industrial application in increasing nitrogen fixation in leguminous plants. The bacterial host may carry the additional DNA fragment encoding the dct genes itself or it may harbour a plasmid carrying such a DNA fragment. The bacterial strain R. meloliti is suitable for use with alfalfa crops, B. japonicum can be used with soya bean crops and R. leguminosarum can be used with pea crops. The DNA inserts encoding the dct genes may be transferred to other strains of bacteria which fix nitrogen. It would be particularly advantageous for such genes to be modified by coupling to a promoter sequence which would lead to even further enhancement of expression.

**Claims**

1. A fragment of DNA encoding genes which complement dicarboxylic acid transport mutants of Rhizobium species, and which confer increased nitrogen fixing ability on plants infected/inoculated with Rhizobium species.

2. A fragment of DNA as claimed in claim I having the restriction data selected from:-

(A)

| Restriction Enzyme | No. of Fragments |
|---|---|
| BglIII | 6 |
| ClaI | 4 |
| EcoRI | 7 |
| HindIII | 4 |
| PstI | 9 |
| SalI | 1 |
| SmaI | 8 |
| BglII/EcoRI | 8 |
| BglII/HindIII | 10 |
| BglII/PstI | 12 |
| BglII/SalI | 6 |
| BglII/SmaI | 11 |

(B)

| Restriction Enzyme | No. of Fragments |
|---|---|
| BamHI | 3 |
| BglII | 7 |
| EcoRI | 7 |
| PstI | 5 |
| HindIII | 4 |

(C)

| Restriction Enzyme | Fragment sizes (kb) |
|---|---|
| BglII | 20.00, 16.60, 8.70, 7.60, 5.90, 1.20 |
| ClaI | 21.00, 13.40, 6.75, 3.45 |
| EcoRI | 21.55, 12.40, 6.15, 5.50, 3.20, 1.55, 1.45 |
| HindIII | 21.00, 12.90, 5.35, 1.75 |
| PstI | 21.00, 5.50, 3.90, 3.20, 2.55, 1.65, 1.55, 1.30, 1.05 |
| SmaI | 18.20, 7.25, 5.50, 5.00, 4.15, 3.35, 2.70, 2.50 |
| BglII/EcoRI | 19.00, 9.35, 5.75, 5.25, 3.80, 2.30, 1.20, 0.85 |
| BglII/HindIII | 20.00, 11.40, 6.05, 4.20*, 2.70, 1.70, 1.20*, 0.85 |
| BglII/PstII | 20.00, 5.50, 3.40, 3.00*, 2.50, 2.05, 1.65*, 1.50, 1.20, 1.05 |
| BglI/SmaI | 15.80, 5.0, 4.20 3.75, 3.40, 3.20, 2.75, 2.50, 1.60*, 7.20 |

*These bands are doublets.

(D)

| Restriction Enzyme | Fragment Sizes (kb) |
|---|---|
| EcoRI | 21.60, 12.75, 3.05, 2.80, 2.60, 1.20, 0.85 |
| BglII | 19.60, 8.90, 8.00, 3.60, 1.95, 1.65, 1.15 |

and fragments of DNA which are substantially similar thereto, particularly having at least 80% homology therewith, and DNA in plasmids deposited with the National Collection of Industrial Bacteria, Torry Research Station, Aberdeen, Scotland under accession numbers NCIB I2293, NCIB I2294 and NCIB I2295 encoding genes which complement dicarboxylic acid transport mutants of Rhizobium species and confer increased nitrogen fixing ability on plants infected/inoculated with Rhizobium species.

3. A plasmid carrying a DNA fragment as claimed in any preceding claim.

4. Plasmids according to claim 3 selected from those deposited with the National Collection of Industrial Bacteria, Torry Research Station, Aberdeen, Scotland under the accession numbers NCIB I2293, NCIB I2294, NCIB I2295, isogenic derivatives threof, plasmids which are substantially similar thereto in the region of DNA encoding genes which complement dicarboxylic acid transport mutants of Rhizobium species, particularly plasmids having at least 80% homology with the said DNA region.

5. Bacterial hosts containing a fragment of DNA as claimed in claim I or 2 or a plasmid as claimed in claim 3 or 4.

6. A fragment of DNA as claimed in claim I or 2, a plasmid as claimed in of claim 3 or 4, or bacterial hosts as claimed in claim 5 in biologically pure form .

7. A method of increasing dicarboxylic acid transport in Rhizobium species comprising transferring into the said species a fragment of DNA as claimed in claim I or 2 or a plasmid as claimed in claim 3 or 4.

8. A method of increasing the nitrogen fixing ability of plants comprising transferring into the said plants a fragment of DNA as claimed in any of claims I, 2 or 6 or a plasmid as claimed in any of claims 3, 4 or 6.

9. Seeds of plants which have been coated/pelleted with bacterial hosts as claimed in claim 5 or claim 6.

FIG.1.

FIG.2.

**FIG.3.**

Rubber seal

Syringe

Sarsted tube containing 2ml 1 NaOH

Assay mixture

**FIG.4.**

nmol succinate/mg protein

CJ1(4:46)

CJ1(4:46:Tn5)

CJ1

CJ1(290)

10

12

8

4

0    2    4    6    8    10

Time (min)

**FIG.5.**

Nitrogenase (nmol $C_2H_4$/mg protein·h)

0.2% malate

0.3% malate

0.4% malate

CJ1(4:46)

CJ1

100

80

60

40

20

0   0.5   1.5   2.5   0.5   1.5   2.5   0.5   1.5   2.5

$pO_2$ (mmHg)

## DOCUMENTS CONSIDERED TO BE RELEVANT

EP 87306660.9

| Category | Citation of document with indication, where appropriate, of relevant passages | Relevant to claim | CLASSIFICATION OF THE APPLICATION (Int. Cl.4) |
|---|---|---|---|
| D,X | ARCHIVES OF MICROBIOLOGY, vol. 144, no. 2, March 1986 (Berlin, Heidelberg, New York) <br><br> E. BOLTON et al. "Dicarboxylic acid transport in Rhizobium meliloti: isolation of mutants and cloning of dicarboxylic acid transport genes" <br> pages 142-146 <br><br> * Totality * <br><br> -- | 1,3,5-8 | C 12 N 15/00 <br> C 07 H 21/04 <br> C 12 N 1/20 <br> A 01 H 1/00 |
| A | EP - A2 - 0 164 992 (AGRIGENETICS RESEARCH ASSOCIATES LIMITED) <br><br> * Claim 1 * <br><br> -- | 1,2,7 | |
| D,A | PROCEEDINGS OF THE NATIONAL ACADEMY OF SCIENCES OF THE UNITED STATES OF AMERICA, vol. 78, no. 7, July 1981 (Baltimore, USA) <br><br> C.W. RONSON et al. "$C_4$-dicarboxylate transport mutants of Rhizobium trifolii form ineffective nodules on Trifolium repens" <br> pages 4284-4288 <br><br> * Totality * <br><br> -- | | TECHNICAL FIELDS SEARCHED (Int. Cl.4) <br><br> C 12 N <br> C 07 H <br> A 01 H |
| D,A | JOURNAL OF BACTERIOLOGY, vol. 137, no. 1, January 1979 (Washington DC) <br><br> M.J. DUNCAN et al. "$\alpha$-Ketoglutarate Dehydrogenase Mutant of Rhizobium meliloti" <br> pages 415-419 <br><br> * Totality * <br><br> ---- | | |

The present search report has been drawn up for all claims

| Place of search | Date of completion of the search | Examiner |
|---|---|---|
| VIENNA | 05-11-1987 | WOLF |